# EUROPÄISCHE PATENTSCHRIFT

(11) **EP 0 744 961 B1**
(45) Veröffentlichungstag und Bekanntmachung des Hinweises auf die Patenterteilung: **19.09.2001**
(21) Anmeldenummer: 95909702.3
(22) Anmeldetag: 10.02.1995
(51) Int. Cl.: A61K 49/00

(54) **GAS ENTHALTENDE MIKROPARTIKEL, DIESE ENTHALTENDE MITTEL, DEREN VERWENDUNG IN DER ULTRASCHALLDIAGNOSTIK, SOWIE VERFAHREN ZUR HERSTELLUNG DER PARTIKEL UND MITTEL**
GAS-CONTAINING MICROPARTICLES, AGENTS CONTAINING THEM, THEIR USE IN MEDICAL DIAGNOSIS BY ULTRASONIC TECHNIQUES AND METHODS OF PRODUCING SAID PARTICLES AND AGENTS
MICROPARTICULES CONTENANT DU GAZ, AGENTS LES CONTENANT, LEUR UTILISATION DANS LES TECHNIQUES DE DIAGNOSTIC PAR ULTRASONS, PROCEDES DE PREPARATION DESDITES PARTICULES ET DESDITS AGENTS

(30) Priorität: 23.02.1994 DE 4406474
(43) Veröffentlichungstag der Anmeldung: 04.12.1996
(60) Teilanmeldung: 98250140.5
(73) Patentinhaber: SCHERING AKTIENGESELLSCHAFT, 13353 Berlin (DE)
(72) Erfinder: HELDMANN, Dieter, D-10555 Berlin (DE); WEITSCHIES, Werner, D-10961 Berlin (DE); FRITZSCH, Thomas, D-12169 Berlin (DE); SPECK, Ulrich, D-13465 Berlin (DE)
(86) Internationale Anmeldenummer: EP9500484
(87) Internationale Veröffentlichungsnummer: WO9522994

(56) Entgegenhaltungen:
- EP-A- 0 122 624
- EP-A- 0 123 235
- EP-A- 0 322 350
- EP-A- 0 365 467
- EP-A- 0 586 875
- WO-A-93/13809

## Beschreibung

Die Erfindung betrifft den in den Patentansprüchen gekennzeichneten Gegenstand, das heißt neue, Gas enthaltende Mikropartikel, diese enthaltende diagnostische Mittel, deren Verwendung in der Ultraschalldiagnostik sowie Verfahren zur Herstellung der Partikel und Mittel.

Seit der Entdeckung durch Gramiak Ende der 60er Jahre, daß Ultraschallkontraste durch Gasbläschen in Flüssigkeiten (Blut) hervorgerufen werden, wurden die verschiedenartigsten Typen gashaltiger Ultraschallkontrastmittel entwickelt und in der Literatur beschrieben.

Der einfachste Typ Ultraschallkontrastmittel läßt sich z.B. durch heftiges Schütteln, durch schnelles Aufziehen und wieder Ausspritzen in (aus) eine(r) Injektionsspritze (sogenanntes "Pumpen") oder Beschallen mit Ultraschall von Lösungen, wie Salzlösungen, Farbstofflösungen oder von vorher entnommenem Blut, erzeugen. Durch die vorbeschriebenen Methoden werden die für die Echokontrastgebung erforderlichen Gasbläschen in das Suspensionsmedium eingebracht. Je nach Wahl des Mediums kann ein mehr oder weniger stabilisierender Effekt des Mediums auf die Mikrogasbläschen erreicht werden.
Derartige Kontrastmittel werden z.B. in der EP 0 077 752 beschrieben. Als Suspensionsmedium finden hier Gemische aus viskositätserhöhender Substanz und Tensid Verwendung. Als Gase werden in der EP 0 077 752 Luft und CO₂ offenbart. Die genannten Kontrastmittel sowie ähnliche, nach den genannten Methoden herstellbare Kontrastmittel, sind mit dem schwerwiegenden Nachteil behaftet, daß die Gasbläschengröße stark variiert und nur schlecht reproduzierbar ist, wodurch ein hohes Embolie-Risiko besteht. Weiterhin lösen sich die durch das Suspensionsmedium nur gering stabilisierten Bläschen schnell auf, so daß ein Kontrasteffekt nur über einen kurzen Zeitraum beobachtet werden kann. Ein Linksherzkontrast nach intravenöser Gabe ist in der Regel bei derartigen Kontrastmittel nicht beobachtbar.

In der WO 93/05819 werden Kontrastmittel auf der Basis von Bläschenemulsionen beschrieben, bei denen jedoch anstelle der herkömmlichen Gase (Luft, Stickstoff, CO₂ und Edelgase), Gase mit einem gewissen Q-Faktor verwendet werden. Bei diesen Gasen handelt es sich in der Regel um halogenierte Kohlenwasserstoffe. Durch die Verwendung dieser konnte der Kontrasteffekt und insbesondere die Signaldauer verlängert werden. Da die Gase auch in diesem Fall - wie schon bei den zuvor beschriebenen Kontrastmitteln - z.B. durch Umpumpen mehrerer Ausgangsstoffe zwischen zwei Spritzen via Dreiwegehahn in das Suspensionsmedium eingebracht werden, zeigen auch diese Mittel eine sehr inhomogene Bläschengrößenverteilung mit dem damit verbundenen Embolie-Risiko.

Die Verwendung bestimmter fluorierter Substanzen als Gase für verschiedene Typen von Ultraschallkontrastmitteln wird auch in der EP 0 554 213 beansprucht. Auch diese Schrift betrifft nicht die erfindungsgemäßen Mikropartikelpräparationen. Das nächstliegende Beispiel (3) der EP 0 554 213 enthält Mikropartikel auf Galactosebasis [wie sie in der EP 0 052 575 / Beispiel 1 beschrieben werden], die anstelle von Luft SF₆ enthalten. Die für diese Partikel beobachteten Effekte sind jedoch schwach und übersteigen die Streuung zwischen den Doppelwerten der Proben nur wenig (siehe Tabelle 3 der EP 0 554 213).

Kontrastmittel mit standardisierter Bläschengröße werden in der EP 0 122 624 und 0 123 235 beschrieben. Diese Kontrastmittel bestehen aus gasenthaltenden Mikropartikeln. Als Partikelmaterial werden Gemische aus grenzflächenaktiven Substanzen wie z.B. Fettsäuren und nicht-grenzflächenaktiven Substanzen wie z.B. Sacchariden verwendet, als Gas wird Luft offenbart. Zwar zeigen diese Mittel die erwünschte Standardisierung bezüglich der Bläschengröße, jedoch lösen sich die Gasbläschen relativ schnell in der Blutflüssigkeit auf, so daß das diagnostische Zeitfenster klein ist.

Ähnliche Kontrastmittel werden in der EP 0 365 467 offenbart. Diese überstehen nach i. v. Applikation des Mittels die Passage des Lungenkapillarbettes und sind somit für die Kontrastierung des linken Herzens und des arteriellen Blutes geeignet. Für einen ausreichend langen und intensiven Kontrasteffekt müssen diese, wie auch die in der EP 0 122 624 und 0 123 235 beschriebenen Kontrastmittel, jedoch in einer Konzentration verabreicht werden, die nicht blutisoton ist, was zu den bekannten Irritationen beim Patienten führen kann.

Weitere Ultraschallkontrastmittel mit standardisierter Bläschengröße werden in der DE 38 03 972 und EP 0 441 468 sowie in der DE 38 03 972 und EP 0 357 163 offenbart. Die beiden erstgenannten Schriften beschreiben Mikropartikel für die Ultraschalldiagnostik bei denen die bildgebenden Substanzen (Gase bzw. niedrig siedende organische Flüssigkeiten) in gekapselter Form vorliegen. Als Hüllmaterial finden Polycyanacrylate (DE 38 03 972) bzw. polymerisierte Aldehyde (EP 0 441 468) Anwendung. Die letztgenannten Schriften beschreiben Mikropartikel bei denen die Gase (bzw. niedrig siedende organische Flüssigkeiten) in komplexierter Form (d.h. in Form eines Wirt/Gast-Komplexes) vorliegen. Obgleich bei diesen Schriften als Gase bzw. niedrig siedende Flüssigkeiten neben den gebräuchlichen Stoffen wie Luft, Stickstoff, auch halogenierte Kohlenwasserstoffe (wie z.B. Brommethan oder Dibromdifluormethan) oder im Falle EP 0 357 163 und EP 0 441 468 auch Schwefelhexafluorid offenbart werden, wird für aus diesen Mikropartikeln bereitete Ultraschallkontrastmittelpräparationen kein bzw. ein nur geringfügiger Einfluß der eingeschlossenen bildgebenden Komponente auf die Kontrastintensität bzw. -dauer beobachtet bzw. beschrieben.

Aufgabe der vorliegenden Erfindung war es daher, ein Kontrastmittel mit definierter Bläschengröße für die Ultraschalldiagnostik bereitzustellen, das in der Lage ist, nach intravenöser Applikation langanhaltende Kontrasteffekte im Blut zu erzielen und dessen Strömungsverhältnisse auf der rechten und linken Herzseite für Ultraschall sichtbar zu machen. Insbesondere sollte durch das Kontrastmittel eine diagnostisch auswertbare Kontrastierung bereits in einem Dosisbereich erreicht werden können, in welchem das Mittel weitgehend blutisoton ist.

Darüber hinaus sollten auch die übrigen an ein in-vivo Kontrastmittel zu stellenden Anforderungen erfüllt sein. Neben einer guten Verträglichkeit ist für ein modernes Kontrastmittel insbesondere ein breites Anwendungsspektrum erwünscht, so sollte es auch für die Darstellung der Durchblutung anderer Organe bzw. Gewebe wie z.B. Myokard, Leber, Milz, Niere und Gehirn, oder nach peroraler oder rektaler Applikation auch für die Darstellung des Gastrointestinaltraktes geeignet sein. Nach Gabe in die Harnblase sollte eine Darstellung des Harnflusses ebenso möglich sein, wie eine Kontrastierung der Tuben nach intrauteriner Applikation. Auch sollte das Kontrastmittel universell in den verschiedenen sonographischen Modi (z.B. B-Mode, Doppler, "Harmonic Imaging") einstetzbar sein.

Diese Aufgabe wird durch die vorliegende Erfindung gelöst.

Es wurde gefunden, daß Mikropartikel bestehend aus der Mischung mindestens einer grenzflächenaktiven Substanz mit mindestens einem nicht-grenzflächenaktiven Stoff und einer bei Körpertemperatur gasförmigen Komponente, dadurch gekennzeichnet, daß als gasförmige Komponente eine Substanz oder ein Substanzgemisch enthalten ist, welche(s) in Wasser schlechter löslich ist (sind) als Luft, das gestellte Anforderungsprofil erfüllen und daher hervorragend als Kontrastmittel in der Ultraschalldiagnostik geeignet sind.

Die durch Suspendieren der erfindungsgemäßen Mikropartikel in einem flüssigen Träger erhaltenen Ultraschallkontrastmittel führen insbesondere zu einer - im Vergleich zum bekannten Stand der Technik - überraschend intensiven und langanhaltenden Kontrastierung. Dadurch kann die für eine Bildgebung erforderliche Dosis gegenüber den in der EP 0 365 467 offenbarten Kontrastmittel um 90% und mehr reduziert werden (siehe auch Beispiel 18). Auf diese Weise können Kontrastmittel erhalten werden, die blutisoton oder nahezu blutisoton sind, wodurch die Verträglichkeit deutlich erhöht wird. Aufgrund der langanhaltenden Kontrasteffekte eignen sich die erfindungsgemäßen Mittel insbesondere auch als "blood pool agents".

Als grenzflächenaktive Substnazen kommen Phospholipide, Sterole, Glycolipide, Saccharoseester wie z.B. Sojasaccharoseglycerid, gesättigte oder ungesättigte Fettsäuren oder deren Salze, Fettalkohole, Mono-, Di- und Triglyceride, Fettsäureester wie z.B. Butylstearat, Xyloglyceride wie z.B. Palmölxylid, polyethoxylierte Sorbitanfettsäureester wie z.B. Polyethylenglycolsorbitanmonostearat, Polyoxyethylenfettsäureester, Polyoxyethylenfettsäureether, Polyoxyethylene, Polyoxyethylen-polyoxypropylen Blockpolymere, ethoxylierte oder sulfatierte Fettalkohole, Alkylarylpolyetheralkohole, fluorierte Fettalkohole, ethoxylierte oder sulfatierte fluorierte Fettalkohole, fluorierte Alkylalkoxylate, wobei gesättigte C₁₂-C₂₆-Fettsäuren, Polyoxyethylen-polyoxypropylen-Blockpolymere und ethoxylierte fluorierte C₁₄-C₃₀-Fettalkohole bevorzugt sind. Die erfindungsgemäßen Mikropartikel enthalten die grenzflächenaktiven Substanzen in einer Konzentration von bis zu 10%, vorzugsweise in einer Konzentration von 0,001 bis 5%, insbesondere in einer Konzentration von 0,01 bis 1%.

Als nicht-grenzflächenaktive Feststoffe kommen sowohl wasserlösliche Feststoffe wie Cyclodextrine, wie z.B. α-,β-, γ-Cyclodextrine, deren monomere, oligomere oder polymere Derivate, Monosaccharidewie z.B. Glucose, Fructose, Galactose, Oligosaccharide wie z.B. Saccharose, Lactose, Maltose, Arabinose, Xylose, Ribose, Polysaccharide wie z.B. Dextran, Stärke oder Stärkederivate, Abbauprodukte von Stärken wie z.B. Dextrine und/oder anorganische oder organische Salze wie z.B. Natriumchlorid, Natriumcitrat, Natriumphosphat, Natriumacetat oder Natriumtatrat sowie Salze triiodierter Benzoesäuren (Amidotrizoat) oder nichtionische Triiodverbindungen (Iopromid), ebenso wie Salze von Komplexen seltener Erden (Gd-DTPA) als auch wasserunlösliche Stoffe wie Tonpartikel, Eisenoxidpartikel oder unlösliche Partikel pflanzlicher Herkunft mit einem Partikeldurchmesser kleiner als 500*µ*m bevorzugt kleiner als 100 *µ*m in Frage. Wobei für die intraveöse Anwendung bevorzugt lösliche Partikel mit einer Partikelgröße kleiner als 10 *µ*m verwendet werden. Besonders bevorzugt für die intravenöse Anwendung sind wasserlösliche Partikel aus Monosacchariden, insbesondere Galaktose oder Disacchariden wie Lactose sowie Partikel aus α- und Hydroxypropyl-β-cyclodextrin. Für perorale oder rektale Applikation finden neben löslichen Partikeln bevorzugt unlösliche Partikel, insbesondere Tonpartikel sowie Partikel pflanzlicher Herkunft Anwendung.

Die erfindungsgemäßen Mikropartikel enthalten die nicht-grenzflächenaktiven Feststoffe in einer Konzentration von mindestens 90, vorzugsweise in einer Konzentration von 95 Gewichtsprozent.

Als bei Körpertemperatur gasförmige halogenierte Verbindungen (nachfolgend kurz als "Gas" bezeichnet) kommen Tetrafluorallene, Hexafluor-1,3-butadien, Dekafluorbutan, Perfluor-1-butene, Perfluor-2-butene, Perfluor-2-butin, Octafluorcyclobutan, Perfluorcyclobuten, Perfluordimethylamin, Hexafluorethan, Tetrafluorethylene, Pentafluorthio(trifluor)methan, Tetrafluormethan, Perfluorpentan, Perfluor-1-penten, Perfluorpropan und/oder Perfluorpropylen in Frage. Erfindungsgemäß bevorzugt sind Hexafluorethan, Dekafluorbutan und/oder Perfluorpropan.

Den genannten fluorierten Gasen können gewünschtenfalls auch bis zu isomolare Mengen an Stickstoff beigemischt werden.

Die erfindungsgemäßen Partikel sind auf vielfältige Weise herstellbar. Sie können z.B. erhalten werden, indem die in der EP 0 365 467, EP 0 122 624, EP 0 123 235 oder in der EP 0 500 023, EP 0 543 020, EP 0 525 199, US 5,107,842 offenbarten Partikel mit den zuvor genannten Gasen behandelt werden, um einen Austausch des in den Partikeln enthaltenen Gases (in der Regel Luft) gegen die gewünschten halogenierten Verbindungen zu erreichen. Dieser Gasaustausch geschieht vorteilhafterweise, indem die jeweiligen Partikel in ein entsprechendes Gefäß eingebracht werden, welches anschließend evakuiert und nachfolgend mit dem gewünschten Gas belüftet wird. Die Inkubation kann auch direkt in den Vials, in denen die Partikel zum Anwender gelangen, erfolgen. Der Gasaustausch kann im Prinzip auch an jeder anderen Stelle des Herstellungsprozesses erfolgen. So besteht insbesondere bei den Partikeln der EP 0 365 467, EP 0 122 624, EP 0 123 235 die Möglichkeit, das Gas während des Zerkleinerungsprozesses, d.h. bei Mahlung z.B. in einer mit dem gewünschten Gas betriebenen "Luftstrahlmühle", einzubringen. Die Atmosphäre des gewünschten Gases ist vorzugsweise bei den weiteren Herstellungsschritten beizubehalten.

Neben dem zuvor beschriebenen nachträglich durchgeführten Gasaustausch kann das Gas aber auch bereits während der Partikelherstellung eingebracht werden. Dabei wird vorteilhafterweise analog zu den in der EP 0 365 467, EP 0 122 624 oder EP 0 123 235 beschriebenen Methoden verfahren, wobei sämtliche Reaktionslösungen zuvor mit dem gewünschten halogenierten Gas gesättigt werden und die gesamte Herstellung unter einer Atmosphäre des gewünschten, halogenierten Gases durchgeführt wird.

Eine Variante des vorbeschriebenen Verfahrens besteht darin, daß zunächst lediglich die nicht-grenzflächenaktive Substanz unter sterilen Bedingungen aus einer mit dem gewünschten Gas gesättigten Lösung rekristallisiert wird. Anschließend wird die grenzflächenaktive Substanz zusammen mit dem nicht-grenzflächenaktiven Feststoff unter sterilen Bedingungen unter der Atmosphäre des gewünschten halogenierten Gases vermischt (agglomeriert) und zerkleinert, bis die gewünschte Partikelgröße von < 10 *µ*m, vorzugsweise < 8 *µ*m, insbesondere 1 - 3 *µ*m erreicht ist. Die Partikelgröße wird in geeigneten Meßgeräten bestimmt.

Ein alternatives Verfahren zur Herstellung der erfindungsgemäßen Mikropartikel besteht darin, die in der EP 0 365 467, EP 0 122 624 oder EP 0 123 235 beschriebenen Partikel in einem geeigneten, mit dem gewünschten halogenierten Gas gesättigten Medium zu lösen und aus diesem zu rekristallisieren. Trocknung, Zerkleinerung, Abfüllung usw. erfolgen wie zuvor beschrieben, wobei sämtliche nachgeschalteten Herstellungsschritte vorteilhafterweise unter einer Atmosphäre des jeweiligen Gases durchgeführt werden.

Aus den erfindungsgemäßen Mikropartikeln lassen sich, durch Suspendieren der Partikel in einem geeigneten physiologisch verträglichem Medium, leicht die erfindungsgemäßen Mittel herstellen. Das Suspendieren erfolgt - insbesondere bei den löslichen Partikeln - vorteilhafterweise erst unmittelbar vor der Injektion durch den behandelnden Arzt, indem aus einem ersten Behälter das Suspensionsmedium unter sterilen Bedingungen, z.B. mittels einer Spritze entnommen, zu den in einem zweiten Behälter befindlichen Mikropartikeln gegeben wird und anschließend durch kurzes (5 bis 10 Sekunden) kräftiges Schütteln der vereinigten Komponenten eine homogene Suspension hergestellt wird. Die erfindungsgemäßen Mittel werden unmittelbar nach ihrer Herstellung, spätestens jedoch innerhalb von 5 Minuten, entweder als Bolus in eine periphere Vene oder in einen zuvor plazierten Katheter, injiziert.

Die Erfindung betrifft daher auch ein Kit für die Herstellung eines Mikropartikel und Gas enthaltenden Ultraschallkontrastmittels bestehend aus einem ersten Behälter, versehen mit einem Verschluß, der die Entnahme des Inhalts unter sterilen Bedingungen ermöglicht und mit dem flüssigen Suspensionsmedium gefüllt ist und einem zweiten Behälter, versehen mit einem Verschluß, der die Zugabe des Suspensionsmediums unter sterilen Bedingungen ermöglicht, gefüllt mit den erfindungsgemäßen Mikropartikeln und einem Gas oder Gasgemisch welches identisch mit dem in den Mikropartikeln enthaltenen Gas ist, wobei das Volumen des zweiten Behälters so bemessen ist, daß das Suspensionsmedium des ersten Behälters vollständig im zweiten behälter Platz findet.
Anstelle zweier separater Behälter kann selbstverständlich auch eine vorgefüllte Spritze bestehend aus zwei Kammern, deren eine Kammer das Suspensionsmedium und deren zweite Kammer die Partikel enthält, verwendet werden.

Als physiologisch verträgliche Suspensionsmedien kommen in Frage Wasser, wäßrige Lösungen eines oder mehrerer anorganischer Salze wie physiologische Kochsalzlösung und Pufferlösungen, wäßrige Lösungen von Mono- oder Disacchariden wie Galactose, Glucose oder Lactose, oder Cyclodextrine, ein- oder mehrwertigen Alkoholen, soweit sie physiologisch verträglich sind, z.B. Ethanol, Polyethylenglykol, Ethylenglykol, Glycerin, Propylenglykol, Propylenglykolmethyl-ester. Bevorzugt sind Wasser und physiologische Elektrolytlösungen wie z.B. physiologische Kochsalzlösung sowie wäßrige Lösungen von Galactose und Glucose. Die Konzentration der gelösten Stoffe beträgt 0,1 bis 30 Gewichtsprozent, vorzugsweise 0,5 bis 25 Gewichtsprozent.

Als Suspensionsmedium für Mikropartikel aus anorganischen Materialien eignen sich die vorgenannten, wobei es sich als vorteilhaft erwiesen hat, dem Medium ein Hydrokolloid wie z.B. Pektin zuzusetzen. Derartige Mittel eignen sich insbesondere zur Kontrastierung des Gastrointestinaltraktes.

Selbstverständlich können den Mitteln unterschiedliche pharmazeutische Hilfsstoffe und Stabilisatoren zugesetzt werden. Auch sind die angegebenen Maße und Prozentwerte als Richtgrößen gedacht. Deren Über- oder Unterschreitung kann im Einzelfall möglich und nützlich sein.

Die erfindungsgemäßen Mittel enthalten je nach Verwendung 5 mg bis 500 mg Partikel pro ml Suspensionsmedium. Die für die Kontrastgebung benötigten Gasbläschen werden durch die Mikropartikel bereitgestellt. Diese sind teilweise an der Oberfläche der Mikropartikel adsorbiert bzw. in den Hohlräumen zwischen den Mikropartikeln oder interkristallin eingeschlossen.

Für intravenöse Anwendungen finden ausschließlich Mittel auf Basis löslicher Partikel Anwendung, für perorale oder rektale Anwendung können darüber hinaus auch Mittel auf Basis unlöslicher Partikel verwendet werden. In Abhängigkeit von der Verwendung variiert auch die jeweils verabreichte Dosis, so werden bei intravenöser Verabreichung in der Regel 0,01 ml bis 1 ml/kg Körpergewicht, bei peroraler Gabe 1 bis 30 ml/kg Körpergewicht appliziert.

Die erfindungsgemäßen Ultraschallkontrastmittel erreichen nach intravenöser Gabe die linke Herzseite und sind somit auch zur Kontrastierung von anderen von der Aorta aus mit Blut versorgten Organen, wie Myokard, Leber, Milz, Niere u.a.m. hervorragend geeignet. Es versteht sich von selbst, daß die erfindungsgemäßen Ultraschallkontrastmittel auch zur Kontrastierung des rechten Herzens und anderer Organe und Körperregionen geeignet sind.

Ein völlig überraschender Vorteil der erfindungsgemäßen Mittel liegt in der Möglichkeit einer außerordentlich starken Dosisreduktion im Vergleich zu den Mitteln des Standes der Technik, wodurch es möglich ist, die Substanzbelastung, die zu applizierenden Volumina bzw. die Osmolarität der Mittel zu senken. Damit können auch Ultraschallkontrastmittel zur Verfügung gestellt werden, die blutisoton oder nahezu blutisoton sind. Ein weiterer Vorteil besteht in der hohen Resistenz gegenüber den eingestrahlten Ultraschallwellen, was zu einer erheblich verbesserten intravitalen Stabilität führt.

Die nachfolgenden Beispiele dienen der näheren Erläuterung des Erfindungsgegenstandes, ohne ihn auf diese beschränken zu wollen.

### Beispiel 1

Mikropartikel hergestellt nach Beispiel 1 der EP 0 365 467 werden in 20 ml Vials zu 2 g abgefüllt. Die Abfüllung erfolgt unter einer Atmosphäre aus Hexafluorethan, welches ebenfalls in den Vials enthalten ist.

### Beispiel 2

Mikropartikel hergestellt nach Beispiel 1 der EP 0 365 467 werden in 20 ml Vials zu 2 g abgefüllt. Die Abfüllung erfolgt unter einer Atmosphäre aus Dekafluorbutan, welches ebenfalls in den Vials enthalten ist.

Perfluorpropan-haltige Partikel können analog zu Beispiel 2 erhalten werden, indem die Abfüllung der Vials unter einer Atmosphäre aus Parfluorbutan erfolgt.

### Beispiel 3

Mikropartikel hergestellt nach Beispiel 3 der EP 0 123 235 werden 24 h in einer Atmosphäre aus Hexafluorethan gelagert (Normaldruck). Die Mikropartikel werden dann unter einer Atmosphäre von Hexafluorethan in Mengen von 2 g in 20 ml Vials abgefüllt.

### Beispiel 4

Mikropartikel hergestellt nach Beispiel 4 der EP 0 123 235 werden 24 h in einer Atmosphäre aus einer isomolaren Mischung aus Hexafluorethan und Dekafluorbutan gelagert. Die Mikropartikel werden dann unter einer isomolaren Atmosphäre aus Hexafluorethan und Dekafluorbutan in Mengen von 2 g in 20 ml Vials abgefüllt.

### Beispiel 5

Mikropartikel hergestellt nach Beispiel 1 der EP 0 365 467 werden in 20 ml Vials zu 3 g abgefüllt. Die Abfüllung erfolgt unter einer Atmosphäre aus Hexafluorethan, welches ebenfalls in den Vials enthalten ist.

### Beispiel 6

Mikropartikel hergestellt nach Beispiel 2 der EP 0 123 235 werden in 20 ml Vials zu 3 g abgefüllt. Die Abfüllung erfolgt unter einer isomolaren Atmosphäre aus Dekafluorbutan und Hexafluorethan, die auch in den Vials enthalten ist.

### Beispiel 7

Mikropartikel hergestellt nach Beispiel 3 der EP 0 123 235 werden in 20 ml Vials zu 3 g abgefüllt. Die Abfüllung erfolgt unter einer Atmosphäre aus Hexafluorethan.

### Beispiel 8

Kontrastmittel nach Beispiel 1 der EP 0 500 023 wurde vor Applikation für 24 h in einer Atmosphäre aus Hexafluorethan inkubiert.

### Beispiel 9

Mikropartikel werden hergestellt nach Beispiel 3 der EP 0 123 235. Die Vermahlung in der Luftstrahlmühle erfolgt jedoch unter Hexafluorethanatmosphäre.

### Beispiel 10

Mikropartikel werden analog zu dem in der EP 0 123 235 / Beispiel 3 beschriebenen Verfahren hergestellt, wobei die Lösungsmittel (Ethanol bzw. Wasser) für die grenzflächenaktive Substanz und die nicht-grenzflächenaktive Substanz zuvor mit Dekafluorbutan übersättigt wurden. Abfüllung und Lagerung erfolgen ebenfalls unter einer Dekafluorbutan-Atmosphäre.

### Beispiel 11

Mikropartikel hergestellt nach Beispiel 1 der EP 0 365 467 werden in geöffneten Vials in einem evakuierbaren Behältnis gelagert und darin bis auf einen Druck von 50 mbar evakuiert. Danach wird das Behältnis mit Hexafluorethan belüftet, die Vials werden unter einer Hexafluorethanatmosphäre verschlossen.

### Beispiel 12

Es wird analog zu Beispiel 11 verfahren, wobei als Gas anstelle von Hexafluorethan Dekafluorbutan eingesetzt wird.

### Beispiel 13

Es wird analog zu Beispiel 11 verfahren, wobei als Gas anstelle von Hexafluorethan Perfluorpropan eingesetzt wird.

### Beispiel 14

Es wird analog zu Beispiel 11 verfahren, wobei als Gas anstelle von Hexafluorethan Perfluorpentan eingesetzt wird.

### Beispiel 15

1997 g Galactose werden in 1080 g Wasser gelöst und auf 5 °C abgekühlt. Zu der entstandenen Suspension werden 3 g Lignocerinsäure, die zuvor in 120 g Ethanol gelöst wurde, unter Rühren zugestetzt. Die Suspension wird anschließend bei 40 °C und einem Unterdruck von 50 mbar getrocknet. Das entstandene Produkt wird mit einer Luftstrahlmühle auf eine Partikelgröße von d_{(99%)} < 8 *µ*m zerkleinert. Die Mikropartikel werden zu einem Granulat agglomeriert und in Portionen zu 2 g je 20 ml Vial abgefüllt, evakuiert, mit Dekafluorbutan begast, 24 Stunden lang in der Dekafluorbutanatmosphäre inkubiert und anschließend verschlossen.

### Beispiel 16 (in vivo Versuch)

4 g einer Präparation hergestellt nach Beispiel 8 werden mit 250 ml einer 1 % igen wäßrigen Pektinlösung als Diluent resuspendiert und peroral einem sedierten Beagle-Hund (11 kg Körpergewicht) verabreicht. (Das Tier wurde innerhalb von 24 Stunden vor dem Versuch nicht gefüttert.) Nach einer intensiven Kontrastierung des Magens wird eine anhaltende Echogenitätserhöhung im Darmlumen erzielt.

### Beispiel 17 (in-vivo Vergleichsbeispiel)

A) 1 g der erfindungsgemäBen Mikropartikel hergestellt nach Beispiel 5 wurde in 2,7 ml Aqua p.i. suspendiert. 2 ml der frisch zubereiteten Suspension wurden einem narkotisierten Beagle-Hund (10 kg Körpergewicht) intravenös injiziert und mit einem Ultraschallgerät das Herz untersucht. Der Kontrastverlauf im linken Ventrikel wurde videodensitometrisch aufgezeichnet und ausgewertet.
B) 1 g Mikropartikel hergestellt nach Beispiel 1 der EP 0 365 467 wurde in 2,7 ml Aqua p.i. suspendiert. 2 ml der frisch zubereiteten Suspension wurden einem narkotisierten Beagle-Hund (10 kg Körpergewicht) intravenös injiziert und mit einem Ultraschallgerät das Herz untersucht. Der Kontrastverlauf im linken Ventrikel wurde videodensitometrisch aufgezeichnet und ausgewertet. Alle übrigen Untersuchungsparameter blieben gegenüber Versuch A) unverändert.

Ergebnis: Die Injektion der erfindungsgemäßen Kontrastmittelpräparation führt zu deutlich intensiverem und deutlich länger anhaltendem Kontrast ("blood pool agent").

### Beispiel 18 (in vivo Vergleichsversuch)

Zu Vergleichszwecken werden Kontrastmittelpräparationen aus
A) Mikropartikeln hergestellt nach Beispiel 1 der EP 0 365 467 und
B) erfindungsgemäßen Mikropartikeln hergestellt nach Beispiel 12 frisch zubereitet. Als Suspensionsmedium wird Wasser p.i. verwendet.

1 ml der jeweiligen Suspensionen werden unmittelbar nach der Zubereitung einem narkotisierten Beagle-Hund (10 kg Körpergewicht) intravenös injiziert und mit einem Ultraschallgerät das Herz untersucht. Der Verlauf der Ultraschallkontrastverstärkung im linken Ventrikel des Herzens wird videodensitometrisch aufgezeichnet und ausgewertet. Die beobachteten Daten (Mittelwerte aus jeweils 3 Versuchen) sind in der nachfolgenden Tabelle zusammengestellt.

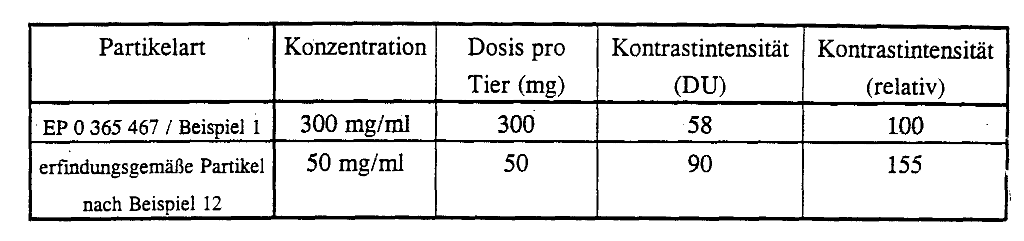

Ergebnis: Die erfindungsgemäßen Kontrastmittel zeigen selbst bei einer Dosisreduktion um den Faktor 6 noch einen intensiveren Kontrast als die Mittel der EP 0 365 467. Von großem Vorteil ist die Blutisotonie der erfindungsgemäßen Präparation im Vergleich zur hypertonen Präparation nach Beispiel 1 der EP 0 365 467.
Von großem Vorteil ist die Blutisotonie der erfindungsgemäßen Präparation im Vergleich zu der hypertonen Präparation nach Beispiel 1 der EP 0 365 467.

### Beispiel 19 (in vivo Vergleichsversuch)

Weitere Daten aus einem analog zu Beispiel 18 durchgeführter Vergleichsversuch sind in nachfolgender Tabelle zusammengefaßt.

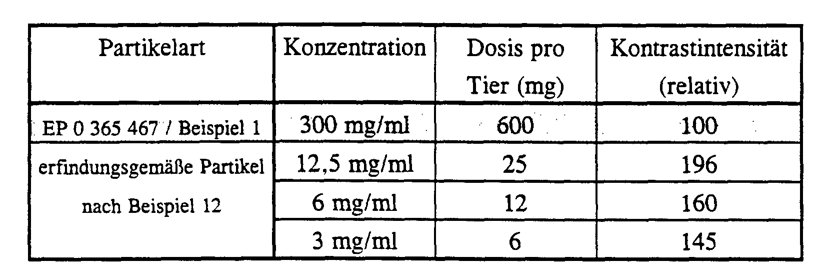

Auch in diesem Fall kann bei Verwendung der erfindungsgemäßen Kontrastmittelpräparation trotz einer Dosisreduktion um bis zum Faktor 100 ein intensiverer Kontrast als bei Verwendung der Kontrastmittelpräparation nach Beispiel 1 der EP 0 365 467 beobachtet werden.

## Patentansprüche

1. Zubereitungen zur Herstellung eines Präparates für die Ultraschalldiagnostik, enthaltend eine bei Körpertemperatur gasförmigen Komponente und Mikropartikel bestehend aus der Mischung mindestens einer grenzflächenaktiven Substanz mit mindestens einem nicht-grenzflächenaktiven Feststoff, **dadurch gekennzeichnet, daß** als bei Körpertemperatur gasförmige Komponente ein halogenierter Kohlenwasserstoff oder ein Gemisch verschiedener halogenierter Kohlenwasserstoffe enthalten ist, welcher (welches) in Wasser schlechter löslich ist als Luft.

2. Mikropartikel nach Anspruch 1, **dadurch gekennzeichnet, daß** als grenzflächenaktive Substanz(en) Phosholipide, Sterole, Glycolipide, ethoxylierte Sojasterine, Polyoxyethylenfettsäureester, ethoxylierte oder sulfatierte Fettalkohole, Polyoxyethylenpolyoxypropylen-Polymere, Saccharoseester, Polyoxyethylenfettsäureether, Polyoxyethylene, gesättigte oder ungesättigte Fettsäuren oder deren Salze, Fettalkohole, Mono-, Di- und Triglyceride, Fettsäureester, Xyloglyceride, Alkylarylpolyetheralkohole, fluorierte Ffettalkohole, ethoxylierte oder sulfatierte fluorieerte Fettalkohole, fluorierte Alkylalkoxylate und/oder polyoxyethoxylierte Sorbitanfettsäureester in einer Menge von 0,01 bis 10 Gewichtsprozent enthalten (sind) ist.

3. Mikropartikel nach Anspruch 1 oder 2, **dadurch gekennzeichnet, daß** als grenzflächenaktive Substanz(en) gesättigte oder ungesättigte C₁₂-C₂₆-Fettsäuren, Polyoxyethylen-polyoxypropylen-Polymere und/oder ethoxylierte fluoorierte Fettalkohole, in einer Konzentration von bis zu 10 Gewichtsprozent enthalten (sind) ist.

4. Mikropartikel nach einem der Ansprüche 1 bis 3, **dadurch gekennzeichnet, daß** als nichtgrenzflächenaktive(r) Feststoff(e) Cyclodextrine, deren Derivate, Monosaccharide, Disaccharide, Trisaccharide, Oligosaccharide, Polysaccharide, Pentosen, Polyole und/oder anorganische oder organische Salze mit einer Konzentration von 90 - 99,999 Gewichtsprozent enthalten (sind) ist.

5. Mikropartikel nach einem der Ansprüche 1 bis 4, **dadurch gekennzeichnet, daß** als nichtgrenzflächenaktive(r) Feststoff(e) Galactose, Lactose, Saccharose, Maltose oder α, β, γ-Cyclodextrine oder deren Derivate in einer Konzentration von 90 - 99,999 Gewichtsprozent enthalten sind.

6. Mikropartikel nach Anspruch 1, **dadurch gekennzeichnet, daß** als nicht grenzflächenaktiver Feststoff Tonpartikel mit einem Partikeldurchmesser kleiner 500 *µ*m enthalten sind.

7. Mikropartikel nach Anspruch 1, **dadurch gekennzeichnet, daß** als nicht grenzflächenaktiver Feststoff unlösliche Partikel pflanzlicher Herkunft enthalten sind.

8. Mikropartikel nach einem der Ansprüche 1 bis 5, **dadurch gekennzeichnet, daß** die Mikropartikel aus einer Mischung von 0,001 bis 10 Gewichtsprozent Butylstearat und 99,999 bis 90 Gewichtsprozent Galactose bestehen.

9. Mikropartikel nach einem der Ansprüche 1 bis 5, **dadurch gekennzeichnet, daß** die Mikropartikel aus einer Mischung von 0,001 bis 10 Gewichtsprozent Sojaölsaccharoseglycerid und 99,999 bis 90 Gewichtsprozent Galactose bestehen.

10. Mikropartikel nach einem der Ansprüche 1 bis 5, **dadurch gekennzeichnet, daß** die Mikropartikel aus einer Mischung von 0,001 bis 10 Gewichtsprozent Polyethylenglycolsorbitanmonostearat und 99,999 bis 90 Gewichtsprozent Galactose bestehen.

11. Mikropartikel nach einem der Ansprüche 1 bis 5, **dadurch gekennzeichnet, daß** die Mikropartikel aus einer Mischung von 0,001 bis 10 Gewichtsprozent Palmölxylit und 99,999 bis 90 Gewichtsprozent Galactose bestehen.

12. Mikropartikel nach einem der Ansprüche 1 bis 11, **dadurch gekennzeichnet, daß** die halogenierte Verbindung mindestens ein Fluoratom enthält.

13. Mikropartikel nach einem der Ansprüche 1 bis 11, **dadurch gekennzeichnet, daß** als halogenierteVerbindung Tetrafluorallene, Hexafluor-1,3-butadien, Dekafluorbutan, Perfluor-1-butene, Perfluor-2-butene, Perfluor-2-butin, Octafluorcyclobutan, Perfluorcyclobuten, Perfluordimethylamin, Hexafluorethan, Tetrafluorethylene, Pentafluorthio(trifluor)methan, Tetrafluormethan, Perfluorpentan, Perfluor-1-penten, Perfluorpropan und/oder Perfluorpropylen enthalten ist (sind).

14. Mikropartikel nach einem der Ansprüche 1 bis 10 oder 12, **dadurch gekennzeichnet, daß** als halogenierte Verbindung Hexafluorethan, Dekafluorbutan und/oder Perfluorpropan enthalten ist (sind).

15. Ultraschallkontrastmittel enthaltend Mikropartikel nach einem der Ansprüche 1 bis 14 in einem physiologisch verträglichen flüssigen Suspensionsmedium, gegebenenfalls mit den in der pharmazeutischen Technologie üblichen Zusätzen.

16. Ultraschallkontrastmittel nach Anspruch 15 enthaltend als physiologisch verträgliches Suspensionsmedium Wasser, physiologische Elektrolytlösung, eine wäßrige Lösung von ein- oder mehrwertigen Alkoholen wie Glycerin, Polyethylenglycol oder von Propylenglykolmethylester oder eine wäßrige Lösung eines Mono- oder Disaccharides.

17. Ultraschallkontrastmittel nach Anspruch 15 enthaltend als Zusatz ein verträgliches Tensid oder ein hydrokolloidales Dispersionsmittel.

18. Ein Kit für die Herstellung eines Mikropartikel und Gas enthaltenenden Ultraschallkontrastmittels bestehend aus
a) einem ersten Behälter, versehen mit einem Verschluß, der die Entnahme des Inhalts unter sterilen Bedingungen ermöglicht und mit dem flüssigen Suspensionsmedium gefüllt ist und
b) einem zweiten Behälter versehen mit einem Verschluß, der die Zugabe des Suspensionsmediums unter sterilen Bedingungen ermöglicht, gefüllt mit Mikropartikeln nach einem der Ansprüche 1 bis 14 und einem Gas oder Gasgemisch welches identisch mit dem in den Mikropartikeln enthaltenen Gas ist, wobei das Volumen des zweiten Behälters so bemessen ist, daß das Suspensionsmedium des ersten Behälters vollständig im zweiten Behälter Platz findet.

19. Verfahren zur Herstellung eines Mikropartikel und Gas enthaltenden Kontrastmittels für die Ultraschalldiagnostik, **dadurch gekennzeichnet, daß** man Mikropartikel nach Anspruch 1 bis 14 mit einer physiologisch verträglichen Trägerflüssigkeit vereinigt und bis zum Entstehen einer homogenen Suspension schüttelt.

20. Verfahren zur Herstellung von Mikropartikeln nach einem der Ansprüche 1 bis 14, **dadurch gekennzeichnet, daß**
a) eine in Wasser gelöste nicht-grenzflächenaktive Substanz unter Zugabe einer alkoholischen Lösung der grenzflächenaktiven Substanz unter Rühren rekristallisiert wird, wobei die jeweiligen Lösungen mit der halogenierten Verbindung gesättigt sind oder
b) Mikropartikel bestehend aus mindestens 90 Gewichtsprozent einer nicht-grenzflächenaktiven Substanz und bis zu 10 Gewichtsprozent einer grenzflächenaktiven Substanz, unter einem Druck von 1 bis 30 Atmosphären 1 Stunde bis zu 6 Tagen mit dem gewünschten halogenierten Gas beschickt werden, wobei vorher gewünschtenfalls andere Gase durch Evakuieren entfernt werden oder
c) Mikropartikel bestehend aus mindestens 90 Gewichtsprozent einer nicht-grenzflächenaktiven Substanz und bis zu 10 Gewichtsprozent einer grenzflächenaktiven Substanz, in einer mit dem halogenierten Gas betriebenen Luftstrahlmühle auf die gewünschte Größe zermahlen werden oder
d) eine nicht-grenzflächenaktive Substanz aus einer mit dem halogenierten Gas gesättigten Lösung rekristallisiert wird und anschließend unter einer Atmosphäre des vorgenannten halogenierten Gases mit der grenzflächenaktiven Substanz vermahlen wird oder
e) Mikropartikel bestehend aus mindestens 90 Gewichtsprozent einer nicht-grenzflächenaktiven Substanz und bis zu 10 Gewichtsprozent einer grenzflächenaktiven Substanz in einem mit dem halogenierten Gas gesättigtem Medium gelöst und anschließend aus diesem rekristallisiert werden.

## Claims

1. Preparations for producing a product for ultrasonic diagnosis, containing a component which is gaseous at body temperature, and microparticles consisting of a mixture of at least one surface-active substance with at least one non-surface-active solid, **characterized in that** a halogenated hydrocarbon or a mixture of various halogenated hydrocarbons, which is less soluble than air in water, is present as component which is gaseous at body temperature.

2. Microparticles according to Claim 1, **characterized in that** the surface-active substance(s) present is(are) phospholipids, sterols, glycolipids, ethoxylated soya sterols, polyoxyethylene fatty acid esters, ethoxylated or sulphated fatty alcohols, polyoxyethylene/polyoxypropylene copolymers, sucrose esters, polyoxyethylene fatty acid ethers, polyoxyethylenes, saturated or unsaturated fatty acids or salts thereof, fatty alcohols, mono-, di- and triglycerides, fatty acid esters, xyloglycerides, alkylaryl polyether alcohols, fluorinated fatty alcohols, ethoxylated or sulphated fluorinated fatty alcohols, fluorinated alkylalkoxylates and/or polyethoxylated sorbitan fatty acid esters in an amount of 0.01 to 10 per cent by weight.

3. Microparticles according to Claim 1 or 2, **characterized in that** the surface-active substance(s) present is(are) saturated or unsaturated C₁₂-C₂₆ fatty acids, polyoxyethylene/polyoxypropylene polymers and/or ethoxylated fluorinated fatty alcohols, in a concentration of up to 10 per cent by weight.

4. Microparticles according to any of Claims 1 to 3, **characterized in that** the non-surface-active solid(s) present is(are) cyclodextrins, derivatives thereof, monosaccharides, disaccharides, trisaccharides, oligosaccharides, polysaccharides, pentoses, polyols and/or inorganic or organic salts with a concentration of 90-99.999 per cent by weight.

5. Microparticles according to any of Claims 1 to 4, **characterized in that** the non-surface-active solid(s) present are galactose, lactose, sucrose, maltose or α,β,γ-cyclodextrins or derivatives thereof in a concentration of 90-99.999 per cent by weight.

6. Microparticles according to Claim 1, **characterized in that** clay particles with a particle diameter of less than 500 *µ*m are present as non-surface-active solid.

7. Microparticles according to Claim 1, **characterized in that** insoluble particles of vegetable origin are present as non-surface-active solid.

8. Microparticles according to any of Claims 1 to 5, **characterized in that** the microparticles consist of a mixture of 0.001 to 10 per cent by weight of butyl stearate and 99.999 to 90 per cent by weight of galactose.

9. Microparticles according to any of Claims 1 to 5, **characterized in that** the microparticles consist of a mixture of 0.001 to 10 per cent by weight of soya oil sucrose glyceride and 99.999 to 90 per cent by weight of galactose.

10. Microparticles according to any of Claims 1 to 5, **characterized in that** the microparticles consist of a mixture of 0.001 to 10 per cent by weight of polyethylene glycol sorbitan monostearate and 99.999 to 90 per cent by weight of galactose.

11. Microparticles according to any of Claims 1 to 5, **characterized in that** the microparticles consist of a mixture of 0.001 to 10 per cent by weight of palm oil xylitol and 99.999 to 90 per cent by weight of galactose.

12. Microparticles according to any one of Claims 1 to 11, **characterized in that** the halogenated compound contains at least one fluorine atom.

13. Microparticles according to any of Claims 1 to 11, **characterized in that** the halogenated compound present is tetrafluorallene, hexafluoro-1,3-butadiene, decafluorobutane, perfluoro-1-butene, perfluoro-2-butene, perfluoro-2-butyne, octafluorocyclobutane, perfluorocyclobutene, perfluorodimethylamine, hexafluoroethane, tetrafluoroethylene, pentafluorothio(trifluoro)methane, tetrafluoromethane, perfluoropentane, perfluoro-1-pentene, perfluoropropane and/or perfluoropropylene.

14. Microparticles according to any of Claims 1 to 10 or 12, **characterized in that** the halogenated compound present is hexafluoroethane, decafluorobutane and/or perfluoropropane.

15. Ultrasonic contrast agent containing microparticles according to any of Claims 1 to 14 in a physiologically tolerated liquid suspending medium, where appropriate with the additives customary in pharmaceutical technology.

16. Ultrasonic contrast agent according to Claim 15 containing as physiologically tolerated suspending medium water, physiological electrolyte solution, an aqueous solution of monohydric or polyhydric alcohols such as glycerol, polyethylene glycol or propylene glycol methyl ether or an aqueous solution of a mono- or disaccharide.

17. Ultrasonic contrast agent according to Claim 15 containing as additive a tolerated surfactant or a hydrocolloidal dispersant.

18. A kit for producing a microparticle- and gas-containing ultrasonic contrast agent consisting of
a) a first container provided with a closure which allows the contents to be removed under sterile conditions and which is filled with the liquid suspending medium, and
b) a second container provided with a closure which allows the suspending medium to be added under sterile conditions, and filled with the microparticles according to any of Claims 1 to 14 and a gas or gas mixture which is identical to the gas present in the microparticles, the volume of the second container being such that there is space in the second container for all the suspending medium in the first container.

19. Process for producing a microparticle- and gas-containing contrast agent for ultrasonic diagnosis, **characterized in that** microparticles according to Claims 1 to 14 are combined with a physiologically tolerated carrier liquid and shaken until a homogeneous suspension is produced.

20. Process for producing microparticles according to any of Claims 1 to 14, **characterized in that**
a) a non-surface-active substance dissolved in water is recrystallized with addition of an alcoholic solution of the surface-active substance with stirring, each of the solutions being saturated with the halogenated compound, or
b) microparticles consisting of at least 90 per cent by weight of a non-surface-active substance and up to 10 per cent by weight of a surface-active substance are charged with the desired halogenated gas under a pressure of 1 to 30 atmospheres for from 1 hour to 6 days, other gases being removed by evacuation beforehand if desired, or
c) microparticles consisting of at least 90 per cent by weight of a non-surface-active substance and up to 10 per cent by weight of a surface-active substance are ground to the desired size in an air-jet mill operated with the halogenated gas, or
d) a non-surface-active substance is recrystallized from a solution saturated with the halogenated gas, and is then ground with the surface-active substance under an atmosphere of the aforementioned halogenated gas, or
e) microparticles consisting of at least 90 per cent by weight of a non-surface-active substance and up to 10 per cent by weight of a surface-active substance are dissolved in a medium saturated with the halogenated gas and then recrystallized therefrom.

## Revendications

1. Préparations pour la fabrication d'une préparation pour diagnostic aux ultrasons, contenant un composant gazeux à la température du corps et des microparticules constituées du mélange d'au moins une substance tensioactive avec au moins un solide non tensioactif, **caractérisées en ce qu'**est contenu comme composant gazeux à la température du corps un hydrocarbure halogéné ou un mélange de divers hydrocarbures halogénés qui est plus difficilement soluble dans l'eau que l'air.

2. Microparticules selon la revendication 1, **caractérisées en ce qu'**est (sont) contenu(s) comme substance(s) tensioactive(s) en quantité de 0,01 à 10 pour cent en poids, des phospholipides, des stérols, des glycolipides, des stérols de soja éthoxylés, des esters d'acides gras polyoxyéthyléniques, des alcools gras éthoxylés ou sulfatés, des polymères de polyoxyéthylène et de polyoxypropylène, des esters de saccharose, des éthers d'acides gras et de polyoxyéthylène, du polyoxyéthylène, des acides gras saturés et insaturés ou leurs sels, des alcools gras, des mono-, di- et triglycérides, des esters d'acides gras, des xyloglycérides, des alkylarylpolyétheralcools, des alcools gras fluorés, des alcools gras fluorés éthoxylés ou sulfatés, des alcoxylates d'alkyle fluorés et/ou des esters d'acides gras de sorbitane polyoxyéthoxylés.

3. Microparticules selon la revendication 1 ou 2, **caractérisées en ce qu'**est (sont) contenu(s) comme substance(s) tensioactive(s) des acides gras en C₁₂-C₂₆ saturés ou insaturés, des polymères de polyoxyéthylène et de polyoxypropylène et/ou des alcools gras fluorés éthoxylés en une concentration allant jusqu'à 10 pour cent en poids.

4. Microparticules selon l'une quelconque des revendications 1 à 3, **caractérisées en ce qu'**est (sont) contenu(e)(s) comme solide(s) non tensioactif(s) des cyclodextrines, leurs dérivés, des monosaccharides, des disaccharides, des trisaccharides, des oligosaccharides, des polysaccharides, des pentoses, des polyols et/ou des sels inorganiques ou organiques en une concentration de 90-99,999 pour cent en poids.

5. Microparticules selon l'une quelconque des revendications 1 à 4, **caractérisées en ce que** sont contenu(e)s comme solide(s) non tensioactif(s) du galactose, du lactose, du saccharose, du maltose ou des α,β,γ-cyclodextrines ou leurs dérivés en concentration de 90-99,999 pour cent en poids.

6. Microparticules selon la revendication 1, **caractérisées en ce que** sont contenues comme solides non tensioactifs des particules d'argile ayant un diamètre particulaire inférieur à 500 *µ*m.

7. Microparticules selon la revendication 1, **caractérisées en ce que** sont contenues comme solide non tensioactif des particules insolubles d'origine végétale.

8. Microparticules selon l'une quelconque des revendications 1 à 5, **caractérisées en ce que** les microparticules sont constituées d'un mélange de 0,001 à 10 pour cent en poids de stéarate de butyle et de 99,999 à 90 pour cent en poids de galactose.

9. Microparticules selon l'une quelconque des revendications 1 à 5, **caractérisées en ce que** les microparticules sont constituées d'un mélange de 0,001 à 10 pour cent en poids de glycéride de saccharose et d'huile de soja et de 99,999 à 90 pour cent en poids de galactose.

10. Microparticules selon l'une quelconque des revendications 1 à 5, **caractérisées en ce que** les microparticules sont constituées d'un mélange de 0,001 à 10 pour cent en poids de monostéarate de polyéthylèneglycolsorbitane et de 99,999 à 90 pour cent en poids de galactose.

11. Microparticules selon l'une quelconque des revendications 1 à 5, **caractérisées en ce que** les microparticules sont constituées d'un mélange de 0,001 à 10 pour cent en poids de xylite d'huile de palme et de 99,999 à 90 pour cent en poids de galactose.

12. Microparticules selon l'une quelconque des revendications 1 à 11, **caractérisées en ce que** le composé halogéné contient au moins un atome de fluor.

13. Microparticules selon l'une quelconque des revendications 1 à 11, **caractérisées en ce qu'**est (sont) contenu(e)(s) comme composé halogéné des tétrafluoroallènes, de l'hexafluoro-1,3-butadiène, du décafluorobutane, des perfluoro-1-butènes, des perfluoro-2-butènes, du perfluoro-2-butyne, de l'octafluorocyclobutane, du perfluorocyclobutène, de la perfluorodiméthylamine, de l'hexafluoroéthane, du tétrafluoroéthylène, du pentafluorothio(trifluoro)méthane, du tétrafluorométhane, du perfluoropentane, du perfluoro-1-pentène, du perfluoropropane et/ou du perfluoropropylène.

14. Microparticules selon l'une quelconque des revendications 1 à 10 ou 12, **caractérisées en ce qu'**est (sont) contenu(s) comme composé halogéné de l'hexafluoroéthane, du décafluorobutane et/ou du perfluoropropane.

15. Agent de contraste aux ultrasons contenant des microparticules selon l'une quelconque des revendications 1 à 14, dans un milieu de suspension liquide physiologiquement compatible, éventuellement avec les additifs habituels en technologie pharmaceutique.

16. Agent de contraste aux ultrasons selon la revendication 15, contenant comme milieu de suspension physiologiquement compatible de l'eau, une solution électrolytique physiologique, une solution aqueuse d'alcools mono- ou polyvalents tels que le glycérol, le polyéthylèneglycol, ou d'ester méthylique de propylèneglycol ou une solution aqueuse d'un mono- ou d'un disaccharide.

17. Agent de contraste aux ultrasons selon la revendication 15, contenant comme additif un agent tensioactif compatible ou un milieu de dispersion hydrocolloïdal.

18. Trousse pour la fabrication d'un agent de contraste aux ultrasons contenant des microparticules et du gaz, constituée :
a) d'un premier récipient pourvu d'une fermeture, qui permet le prélèvement du contenu dans des conditions stériles et est rempli du milieu de suspension liquide, et
b) d'un second récipient pourvu d'une fermeture, qui permet l'addition du milieu de suspension dans des conditions stériles, rempli de microparticules selon l'une quelconque des revendications 1 à 14 et d'un gaz ou d'un mélange de gaz qui est identique au gaz contenu dans les microparticules, le volume du second récipient étant dimensionné de telle sorte que le milieu de suspension du premier récipient prenne place intégralement dans le second récipient.

19. Procédé de fabrication d'un agent de contraste contenant des microparticules et du gaz pour diagnostic aux ultrasons, **caractérisé en ce que** l'on combine des microparticules selon les revendications 1 à 14 avec un liquide support physiologiquement compatible et on secoue le tout jusqu'à la formation d'une suspension homogène.

20. Procédé de fabrication de microparticules selon l'une quelconque des revendications 1 à 14, **caractérisé en ce que** :
a) on recristallise sous agitation une substance non tensioactive dissoute dans de l'eau en ajoutant une solution alcoolique de la substance tensioactive, les solutions respectives étant saturées du composé halogéné, ou
b) des microparticules constituées d'au moins 90 pour cent en poids d'une substance non tensioactive et d'une quantité jusqu'à 10 pour cent en poids d'une substance tensioactive sont alimentées, sous une pression de 1 à 30 atmosphères, pendant 1 heure à 6 jours, en le gaz halogéné souhaité, les autres gaz étant éventuellement éliminés préalablement par mise sous vide, ou
c) des microparticules constituées d'au moins 90 pour cent en poids d'une substance non tensioactive et d'une quantité jusqu'à 10 pour cent en poids d'une substance tensioactive sont micronisées à la taille souhaitée dans un broyeur à jet d'air fonctionnant avec le gaz halogéné, ou
d) une substance non tensioactive est recristallisée à partir d'une solution saturée par le gaz halogéné et ensuite broyée sous une atmosphère du gaz halogéné précité avec la substance tensioactive, ou
e) des microparticules constituées d'au moins 90 pour cent en poids d'une substance non tensioactive et d'une quantité jusqu'à 10 pour cent en poids d'une substance tensioactive sont dissoutes dans un milieu saturé par le gaz halogéné et ensuite recristallisées à partir de ce milieu.
